(19) Europäisches Patentamt
European Patent Office
Office européen des brevets

(11) Numéro de publication: **0 063 066 B1**

# (12) FASCICULE DE BREVET EUROPEEN

(45) Date de publication du fascicule du brevet: **20.06.84**

(51) Int. Cl.³: **C 07 C 25/02**, C 07 C 25/13, C 07 C 17/00

(21) Numéro de dépôt: **82400549.0**

(22) Date de dépôt: **26.03.82**

## (54) Procédé d'isomérisation de bromohalogénobenzènes.

(30) Priorité: **08.04.81 FR 8107026**

(43) Date de publication de la demande: **20.10.82 Bulletin 82/42**

(45) Mention de la délivrance du brevet: **20.06.84 Bulletin 84/25**

(84) Etats contractants désignés: **AT BE CH DE FR GB IT LI LU NL SE**

(56) Documents cités:
**FR - A - 2 362 807**

**JOURNAL OF THE AMERICAN CHEMICAL SOCIETY, vol. 85, 20 juin 1963, EASTON (US) C.E. MOYER et al. "Base-catalyzed isomerization of trihalobenzenes", pages 1891-1893**

(73) Titulaire: **RHONE-POULENC SPECIALITES CHIMIQUES, "Les Miroirs" 18, Avenue d'Alsace, F-92400 Courbevoie (FR)**

(72) Inventeur: **Soula, Gérard, 33, rue Nungesser, F-69330 - Meyzieu (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 063 066 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé d'isomérisation de bromohalogénobenzènes.

Ce type de réaction est connu dans l'art antérieur.

C'est ainsi que la demande de brevet français 7 725 834 publiée sous le numéro 2 362 807 décrit la préparation de bromo-1 dichloro-3,5 benzène par isomérisation du bromo-1 dichloro-2,4 benzène à 80–180°C en présence d'un halogénure d'aluminium comme $AlCl_3$ anhydre. Ce procédé présente plusieurs inconvénients qui rendent délicate sa mise en œuvre industrielle. En effet, en premier lieu, le taux d'isomérisation est limité. En second lieu, on assiste à la formation de nombreux isomères du produit recherché: les dichloro-2,3 bromobenzène, dichloro-2,5 bromobenzène, dichloro-3,4 bromobenzène et dichloro-2,6 bromobenzène. Or le produit industriellement intéressant est le dichloro-3,5 bromobenzène qui permet notamment d'avoir accès à la dichloro-3,5 aniline. En troisième lieu, il est à noter qu'on observe, dans le procédé de cette demande, la formation de produits de dismutation (dichlorobenzènes et dichlorodibromobenzènes). On est en présence, en fin de réaction, de mélanges très complexes qui nécessitent des opérations de séparation longues et coûteuses. Un dernier inconvénient à relever est la température élevée qu'il est nécessaire de maintenir pour pouvoir mener à bien la réaction.

On connaît aussi l'article de Moyer et Bunnett dans J.A.C.S. Vol. 85 (1963) pages 1891–1893 qui décrit l'obtention de bromo-1 dichloro-3,5 benzène à partir de bromo-1 dichloro-2,4 benzène en présence d'anilide de potassium dans l'ammoniac liquide. Les rendements obtenus selon ce procédé sont très faibles (33%) et interdisent dans la pratique toute exploitation industrielle.

On voit donc que subsiste dans l'art antérieur, le besoin d'un procédé d'isomérisation de bromohalogénobenzènes qui permet l'obtention sélective du produit recherché dans des conditions de température douces et la récupération aisée du produit formé tout en ne mettant pas en œuvre des solvants dont la manipulation est industriellement délicate.

La présente invention atteint cet objectif.

La présente invention a donc pour objet un procédé d'isomérisation de bromohalogénobenzènes caractérisé en ce que l'on met en présence un bromohalogénobenzène de formule:

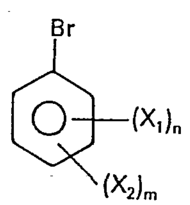

où $X_1$ et $X_2$ identiques ou différents représentent un atome de chlore ou de fluor

n et m sont supérieurs ou égaux à 0 et inférieurs ou égaux à 3, la somme n + m étant égale à 2 ou 3, une base alcaline et au moins un composé complexant le cation de la base alcaline.

Selon un premier mode de mise en œuvre particulier de l'invention, le composé complexant le cation de la base alcaline est un polyéther macrocyclique ayant de 15 à 30 atomes dans le cycle et constitué de 5 à 10 unités -O-X dans lesquelles X est soit $-CHR_1-CHR_2-$ soit $-CHR_1-CHR_4-CR_3R_2-$, $R_1$, $R_2$, $R_3$ et $R_4$ identiques ou différents étant un atome d'hydrogène ou un radical alkyle ayant de 1 à 4 atomes de carbone, un des X pouvant être $-CHR_1-CHR_4-CR_3-R_2-$ quand les unités -O-X comprennent le groupement $-O-CHR_1-CHR_2-$.

Selon un deuxième mode de mise en œuvre particulier de l'invention, le composé complexant le cation de la base alcaline est un composé macrocyclique ou bicyclique de formule générale Ia ou Ib

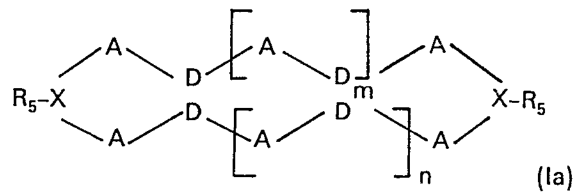

(Ia)

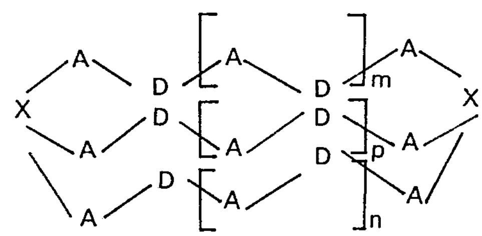

(Ib)

dans lesquelles:

X représente N ou P

A représente un groupement alkylène ayant de 1 à 3 atomes de carbone

D représente O, S ou $N-R_6$ où $R_6$ représente un radical alkyle ayant de 1 à 6 atomes de carbone

$R_5$ représente un radical alkyle ayant de 1 à 6 atomes de carbone, n, m et p identiques ou différents sont des nombres entiers compris entre 1 et 5.

Selon un troisième mode de mise en œuvre particulier de l'invention, le composé complexant le cation de la base alcaline est une amine de formule générale

$$N-[CHR_7-CHR_8-O-(CHR_9-CHR_{10}-O)_s-R_{11}]_3 \quad (II)$$

dans laquelle s est un nombre entier de préférence supérieur ou égal à 0 et inférieur ou égal à 10 ($0 \leqq s \leqq 10$), $R_7$, $R_8$, $R_9$ et $R_{10}$ identiques ou différents représentent un atome d'hydrogène ou un radical alkyle ayant de 1 à 4 atomes de carbone et $R_{11}$ représente un radical alkyle ou cycloalkyle ayant de 1 à 12 atomes de carbone, un radical phényle ou un radical $-C_qH_{2q}-\varnothing$ ou $C_qH_{2q+1}-\varnothing-$, où q est compris entre 1 et 12.

Selon un quatrième mode de mise en œuvre particulier de l'invention, le composé complexant le cation de la base alcaline est un polyéther linéaire de formule générale:

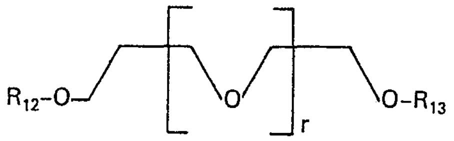

(III)

dans laquelle r est de préférence compris entre 1 et 10 et où $R_{12}$ et $R_{13}$ identiques ou différents représentent un radical alkyle ayant de 1 à 12 atomes de carbone.

Les polyéthers macrocycliques qui peuvent être mis en œuvre dans le procédé selon l'invention sont connus sous l'appellation générale d'«éthers couronnes» et sont décrits dans le brevet français 6 943 879 publié sous le numéro 2 026 481.

On peut citer comme exemples d'éthers couronnes pouvant être utilisés selon l'invention:

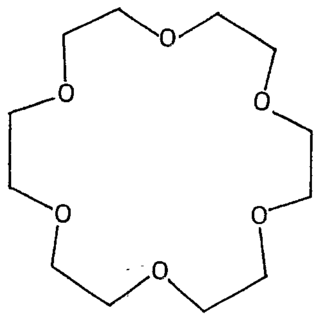

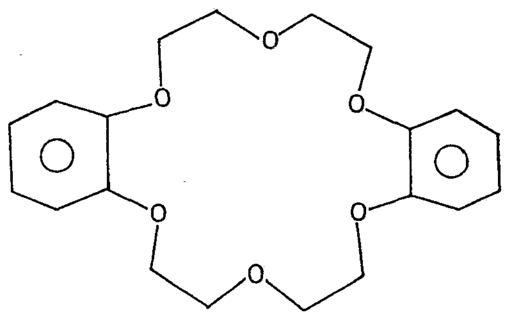

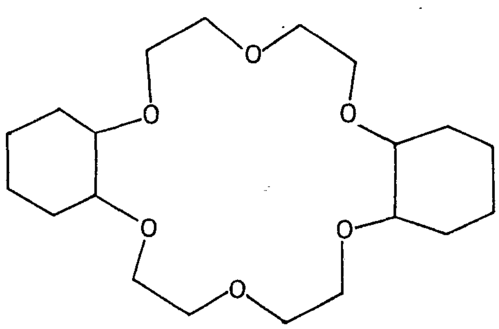

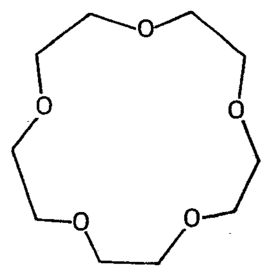

Les composés macrocycliques et bicycliques de formules générales Ia et Ib sont décrits dans le brevet français 7 021 079 publié sous le numéro 2 052 947. On peut citer comme exemples de tels composés convenant pour la mise en œuvre du procédé selon l'invention:

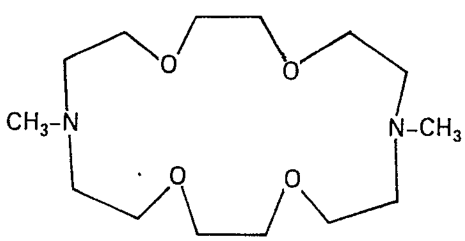

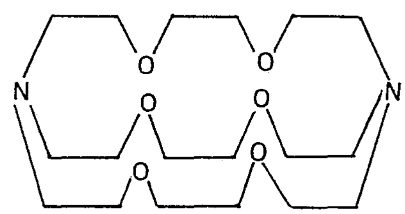

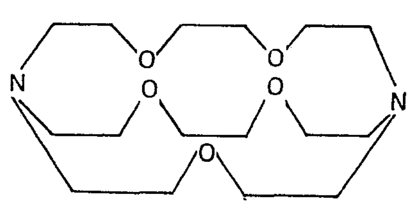

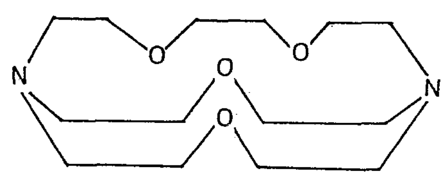

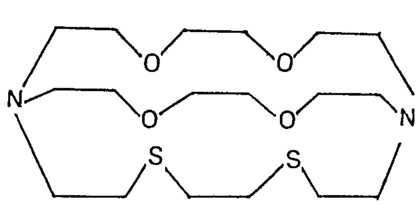

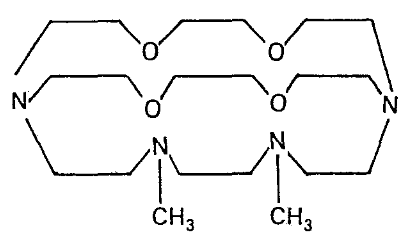

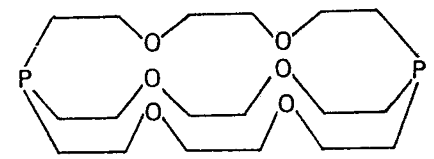

Les amines de formule générale II sont décrites dans la demande de brevet français 7 905 438 publiée sous le numéro 2 450 120.

Les amines de formule II préférées sont celles pour lesquelles $R_7$, $R_8$, $R_9$ et $R_{10}$ représentent n atome d'hydrogène ou un radical méthyle, $R_{11}$ et s ayant la signification précédente.

On préfère encore plus particulièrement celles pour lesquelles s est supérieur ou égal à 0 et inférieur ou égal à 6 et $R_{11}$ représente un radical alkyle ayant de 1 à 4 atomes de carbone.

On peut citer comme exemples d'amines de formule II pouvant être utilisées dans le procédé de l'invention les composés suivants:

la tris(oxa-3 heptyl)amine de formule:
$N-(CH_2-CH_2-O-C_4H_9)_3$
la tris(dioxa-3,6 heptyl)amine de formule:
$N-(CH_2-CH_2-O-CH_2-CH_2-O-CH_3)_3$
la tris(trioxa-3,6,9 décyl)amine de formule:
$N-(CH_2-CH_2-O-CH_2-CH_2-O-CH_2-CH_2-O-CH_3)_3$
la tris(dioxa-3,6 octyl)amine de formule:
$N-(CH_2-CH_2-O-CH_2-CH_2-O-C_2H_5)$
la tris(trioxa-3,6,9 undécyl)amine de formule:
$N-(CH_2-CH_2-O-CH_2-CH_2-O-CH_2-CH_2-O-C_2H_5)_3$
la tris(dioxa-3,6 nonyl)amine de formule:
$N-(CH_2-CH_2-O-CH_2-CH_2-O-C_3H_7)$
la tris(trioxa-3,6,9 dodécyl)amine de formule:
$N-(CH_2-CH_2-O-CH_2-CH_2-O-CH_2-CH_2-O-C_3H_7)_3$
la tris(dioxa-3,6 décyl)amine de formule:
$N-(CH_2-CH_2-O-CH_2-CH_2-O-C_4H_9)$
la tris(trioxa-3,6,9 tridécyl)amine de formule:
$N-(CH_2-CH_2-O-CH_2-CH_2-O-CH_2-CH_2-O-C_4H_9)_3$
On peut encore citer:
la tris(dioxa-3,6 méthyl-4 heptyl)amine de formule:

$$N-(CH_2-CH_2-O-\underset{\displaystyle CH_3}{\underset{|}{CH}}-CH_2-O-CH_3)_3$$

la tris(dioxa-3,6 diméthyl-2,4 heptyl)amine de formule:

$$N-(CH_2-\underset{\displaystyle CH_3}{\underset{|}{CH}}-O-\underset{\displaystyle CH_3}{\underset{|}{CH}}-CH_2-O-CH_3)_3$$

Les polyéthers de formule III préférés sont ceux pour lesquels n est compris entre 1 et 4 et $R_{12}$ représente un radical alkyle ayant de 1 à 6 atomes de carbone.

On peut citer comme exemples de composés de formule III pouvant être mis en œuvre dans le cadre de la présente invention:

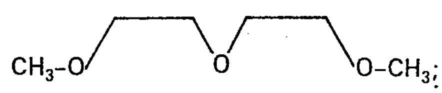 ; 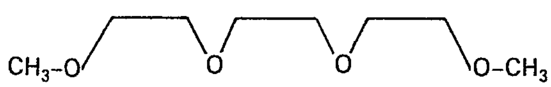

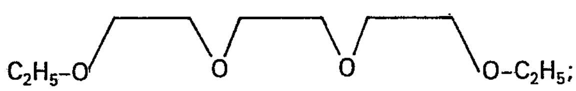 ; 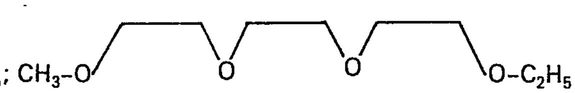

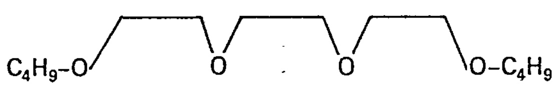

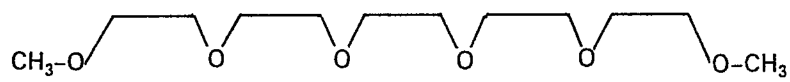

On peut utiliser les divers composés ci-dessus seuls ou en mélange.

La base alcaline mise en œuvre dans le procédé de l'invention est choisie de préférence parmi les amidures alcalins. On préfère tout particulièrement l'amidure de sodium, de potassium ou de lithium.

On peut citer comme bromohalogénobenzènes pouvant être mis en œuvre dans le procédé selon l'invention les composés suivants (sont mentionnés entre parenthèses les composés obtenus):

dichloro-2,4 bromobenzène (dichloro-3,5 bromobenzène), dichloro-2,3 bromobenzène (dichloro-3,4 bromobenzène), trichloro-2,3,4 bromobenzène (trichloro-3,4,5 bromobenzène), difluoro-2,4 bromobenzène (difluoro-3,5 bromobenzène), difluoro-2,3 bromobenzène (difluoro-3,4 bromobenzène), difluoro-2,4 chloro-3 bromobenzène (difluoro-3,5 chloro-4 bromobenzène), trifluoro-2,3,4 bromobenzène (trifluoro-3,4,5 bromobenzène).

L'invention est particulièrement intéressante compte tenu des produits formés, dans le cas où le produit de départ est le dichloro-2,4 bromobenzène, le dichloro-2,3 bromobenzène ou le difluoro-2,4 bromobenzène.

On peut opérer en présence ou en l'absence de solvant. Dans le premier cas, on utilise de préférence un solvant aprotique apolaire ou aprotique peu polaire comme, par exemple, le toluène, le chlorobenzène, le tétrahydrofurane, le dioxanne ou l'éther diméthylique de l'éthylène glycol.

On opère de préférence à une température

comprise entre 0 et 60°C et plus particulièrement entre 10 et 40°C.

On utilise la base alcaline en quantité telle que le rapport molaire de la base alcaline au bromohalogénobenzène est compris de préférence entre 0,05 et 1. Encore plus préférentiellement ce rapport est compris entre 0,05 et 0,5.

Le rapport molaire du composé complexant le cation de la base alcaline au bromohalogénobenzène est compris de préférence entre 0,005 et 0,2. Encore plus préférentiellement, il est compris entre 0,01 et 0,1.

Le procédé selon l'invention est mis en œuvre à la pression atmosphérique bien que des pressions supérieures ou inférieures à la pressoin atmosphérique ne soient pas exclues du domaine de l'invention.

La présente invention sera plus complètement décrite à l'aide des exemples ci-après qui ne sauraient être considérés comme limitant sa portée.

Exemple 1

Isomérisation du dichloro-2,4 bromobenzène

Dans un réacteur tétracol de 500 ml équipé d'un agitateur mécanique, d'un thermomètre, d'une arrivée d'azote et d'une ampoule à brome, équipée elle-même d'un agitateur mécanique, on introduit successivement sous courant d'azote 169 g de chlorobenzène anhydre, 113 g de dichloro-2,4 bromobenzène (0,5 mole) et 8,1 g de tris(dioxa-3,6 heptyl)amine (0,025 mole). Le mélange étant agité, on ajoute alors en 1 heure 6 g d'amidure de sodium en suspension dans 6 g de toluène à l'aide de l'ampoule à brome. On maintient l'agitation pendant 1 heure après la fin de l'addition de l'amidure puis on ajoute 8 g de chlorure d'ammonium pour neutraliser l'amidure de sodium.

Le mélange est ensuite lavé avec 100 $cm^3$ d'une solution d'acide chlorhydrique à 10% puis décanté et séché sur silicagel.

Le chlorobenzène est éliminé et les produits sont distillés. On récupère ainsi 2,6 g de méta dichlorobenzène, 97 g de dichlorobromobenzènes composés de 78% de dichloro-3,5 bromobenzène et de 22% de dichloro-2,4 bromobenzène.

Exemples 2 à 5

Isomérisation du dichloro-2,4 bromobenzène

Dans un réacteur de 20 ml équipé d'un condenseur et placé sous courant d'azote, on introduit successivement 1,6 g de chlorobenzène, 1,13 g de dichloro-2,4 bromobenzène (5 mmoles), 0,1 g d'amidure de sodium (1,3 mmole) en suspension dans 0,1 g de toluène et un catalyseur dont la concentration varie entre 5 et 10% molaire. Le mélange est agité à 20°C pendant un temps déterminé puis analysé par chromatographie en phase gazeuse. Les résultats obtenus selon le catalyseur employé et le temps réactionnel sont rassemblés dans le tableau I.

Essai comparatif

On opère comme ci-dessus mais sans ajouter de catalyseur. Après 3 heures le taux de transformation est nul.

Exemple 6

Isomérisation du dichloro-2,3 bromobenzène

Dans un réacteur de 50 ml équipé d'un condenseur et placé sous courant d'azote, on introduit successivement 6,8 g de chlorobenzène, 2,26 g de dichloro-2,3 bromobenzène (0,01 mole), 0,22 g d'amidure de sodium dispersé dans 0,22 g de toluène et 0,32 g de tris(dioxa-3,6 hexyl)amine (0,0028 mole). Le mélange est agité pendant 2 h à 21°C puis analysé par chromatographie en phase gazeuse. Le taux d'isomérisation du dichloro-2,3 bromobenzène en dichloro-3,4 bromobenzène est de 42% et le rendement de la réaction est de 92%.

Tableau 1

| Ex | Catalyseur (% molaire) | Temps réactionnel | Taux de transformation dichloro-3,5 bromo benzène (%) | Rdt (%) |
|---|---|---|---|---|
| 2 | Cryptand (2,2,2) (*), (5%) | 30 min | 84 | 70 |
| 3 | Ether-couronne (**), dicyclohexyl 18 C.6, (5%) | 30 min | 82 | 94 |
| 4 | Diméthyl éther du triéthylène glycol, (10%) | 3 h | 70 | 90 |
| 5 | Tris(dioxa-3,6 heptyl)amine, (5%) | 1 h | 80 | 94 |

(*) 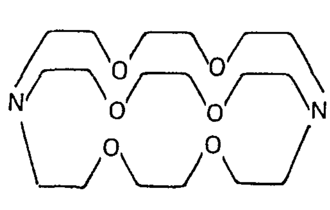

(**) 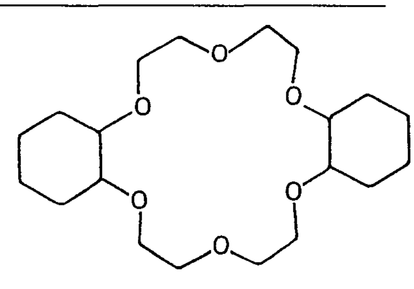

Exemple 7

Isomérisation du difluoro-2,4 bromobenzène

Dans un réacteur de 50 ml équipé d'un réfrigérant et d'un agitateur magnétique, on introduit successivement 19,3 g de difluoro-2,4 bromobenzène (0,1 mole), 4,6 g de toluène et 1,6 g de tris(dioxa-3,6 heptyl) amine (0,005 mole). Le mélange est refroidi à 10°C sous azote puis on ajoute 0,19 g d'amidure de sodium (0,005 mole) en suspension dans 0,2 g de toluène. Après 20 minutes de réaction sous agitation le mélange obtenu se compose de 79% de difluoro-3,5 bromobenzène et de 21% de difluoro-2,4 bromobenzène. Le mélange est distillé. On récupère alors 12,1 g de difluoro-3,5 bromobenzène ($\theta = 140°C$).

**Revendications**

1. Procédé d'isomérisation de bromohalogénobenzènes caractérisé en ce que l'on met en présence un bromohalogénobenzène de formule:

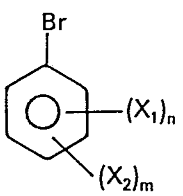

où $X_1$ et $X_2$ identiques ou différents représentent un atome de chlore ou de fluor
n et m sont supérieurs ou égaux à 0 et inférieurs ou égaux à 3, la somme n + m étant égale à 2 ou 3, une base alcaline et au moins un composé complexant le cation de la base alcaline.

2. Procédé selon la revendication 1 caractérisé en ce que le composé complexant le cation de la base alcaline est un polyéther macrocyclique ayant de 15 à 30 atomes dans le cycle et constitué de 5 à 10 unités -O-X dans lesquelles X est soit $-CHR_1-CHR_2-$ soit $-CHR_1-CHR_4-CR_3R_2-$, $R_1$, $R_2$, $R_3$ et $R_4$ identiques ou différents étant un atome d'hydrogène ou un radical alkyle ayant de 1 à 4 atomes de carbone, un des X pouvant être $-CHR_1-CHR_4-CR_3R_2-$ quand les unités -O-X comprennent le groupement $-O-CHR_1-CHR_2-$.

3. Procédé selon la revendication 1 caractérisé en ce que le composé complexant le cation de la base alcaline est un composé macrocyclique ou bicyclique de formule générale Ia ou Ib

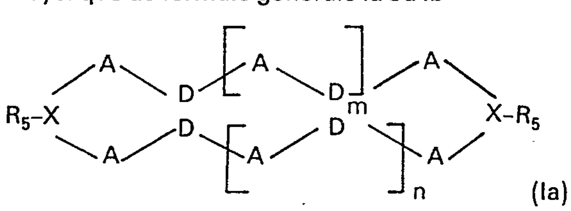

(Ia)

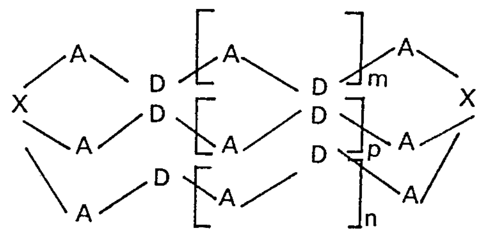

(Ib)

dans lesquelles:

X représente N ou P
A représente un groupement alkylène ayant de 1 à 3 atomes de carbone
D représente O, S ou $N-R_6$ où $R_6$ représent un radical alkyle ayant de 1 à 6 atomes de carbone
$R_5$ représente un radical alkyle ayant de 1 à 6 atomes de carbone, n, m et p identiques ou différents sont des nombres entiers compris entre 1 et 5.

4. Procédé selon la revendication 1 caractérisé en ce que le composé complexant le cation de la base alcaline est une amine de formule générale:

$$N-[CHR_7-CHR_8-O-(CHR_9-CHR_{10}-O)_s-R_{11}]_3 \quad (II)$$

dans laquelle s est un nombre entier supérieur ou égal à 0 et inférieur ou égal à 10 ($0 \leq s \leq 10$), $R_7$, $R_8$, $R_9$ et $R_{10}$ identiques ou différents représentent un atome d'hydrogène ou un radical alkyle ayant de 1 à 4 atomes de carbone et $R_{11}$ représente un radical alkyle ou cycloalkyle ayant de 1 à 12 atomes de carbone, un radical phényle ou un radical $-C_qH_{2q}-\varnothing$ ou $C_qH_{2q+1}-\varnothing-$, où q est compris entre 1 et 12.

5. Procédé selon la revendication 1 caractérisé en ce que le composé complexant le cation de la base alcaline est un polyéther linéaire de formule générale (III)

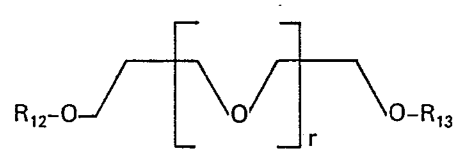

(III)

dans laquelle r est compris entre environ 1 et environ 10 et ou $R_{12}$ et $R_{13}$ identiques ou différents représentent un radical alkyle ayant de 1 à 12 atomes de carbone.

6. Procédé selon la revendication 2 caractérisé en ce que le polyéther macrocyclique est choisi parmi le groupe comprenant:

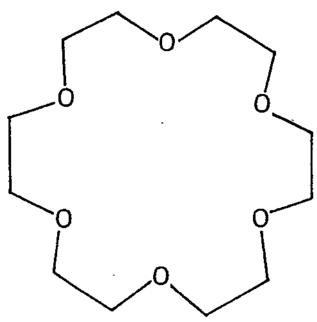

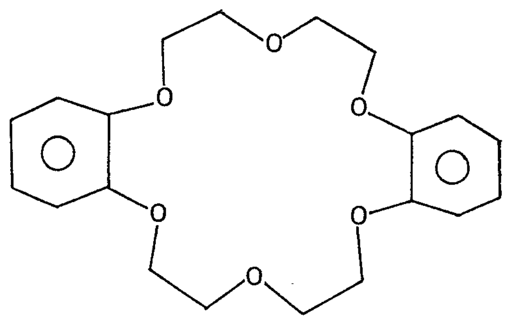

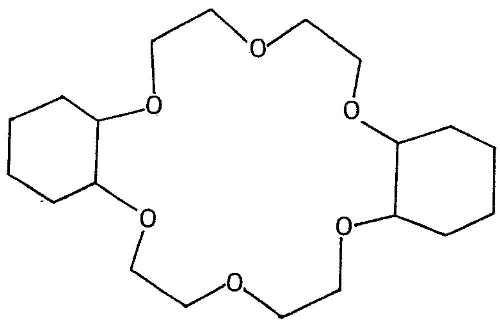

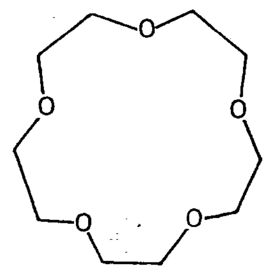

7. Procédé selon la revendication 3 caractérisé en ce que le composé macrocyclique ou bicyclique est choisi parmi le groupe comprenant:

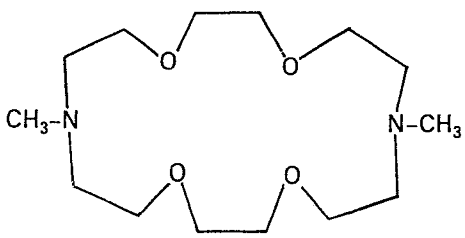

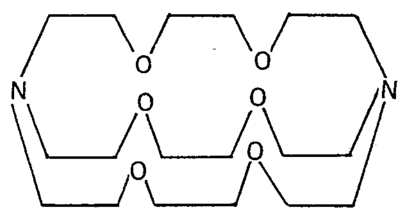

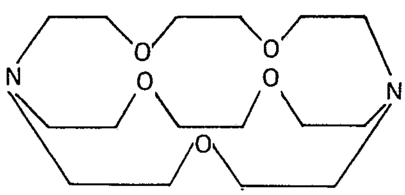

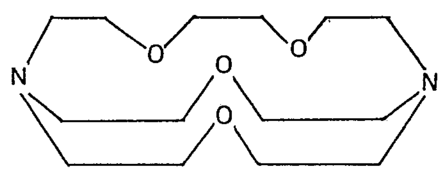

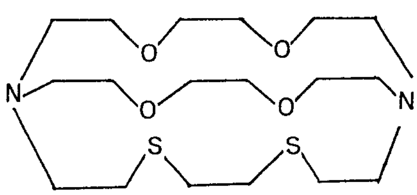

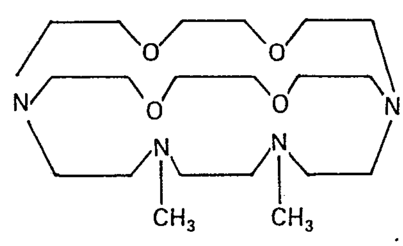

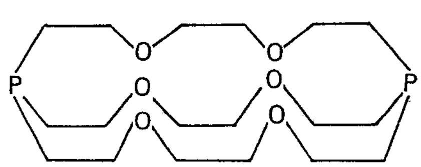

8. Procédé selon la revendication 4 caractérisé en ce que dans la formule II, $R_7$, $R_8$, $R_9$ et $R_{10}$ représentent un atome d'hydrogène ou un radical méthyle.

9. Procédé selon la revendication 4 caractérisé en ce que dans la formule II, s est supérieur ou égal à 0 et inférieur ou égal à 6 et $R_{11}$ représente un radical alkyle ayant de 1 à 4 atomes de carbone.

10. Procédé selon les revendications 8 et 9 caractérisé en ce que l'amine est choisie parmi le groupe comprenant la tris(dioxa-3,6 heptyl)amine; la tris (dioxa-3,6 hexyl)amine.

11. Procédé selon la revendication 5 caractérisé en ce que le polyéther linéaire est choisi parmi le groupe comprenant

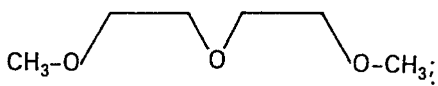
;
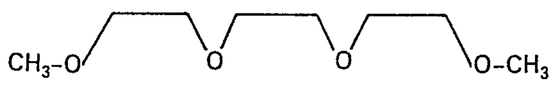

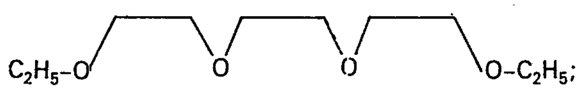
;
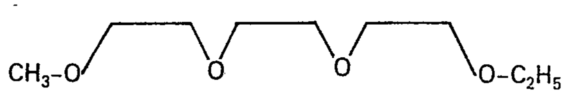

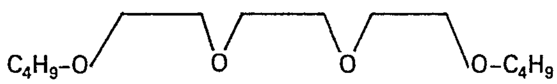

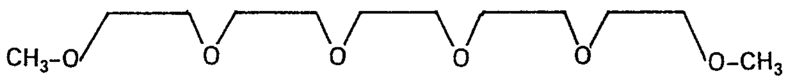

12. Procédé selon l'une quelconque des revendications principales caractérisé en ce que la base alcaline est un amidure alcalin.

13. Procédé selon l'une quelconque des revendications précédentes caractérisé en ce que le bromohalogénobenzène mis en œuvre est le dichloro-2,4 bromobenzène, le dichloro-2,3 bromobenzène ou le difluoro-3,5 bromobenzène.

14. Procédé selon l'une quelconque des revendications précédentes caractérisé en ce qu'on opère en présence d'un solvant aprotique apolaire ou aprotique peu polaire.

15. Procédé selon l'une quelconque des revendications précédentes caractérisé en ce que le rapport molaire de la base alcaline au bromohalogénobenzène est compris entre 0,05 et 1.

16. Procédé selon l'une quelconque des revendications précédents caractérisé en ce que le rapport molaire du composé complexant le cation de la base alcaline est compris entre 0,005 et 0,2.

17. Procédé selon l'une quelconque des revendications précédentes caractérisé en ce que l'on opère à une température comprise entre 0 et 60°C.

**Claims**

1. A process for isomerising bromohalobenzenes, characterised by bringing together a bromohalobenzene of the formula:

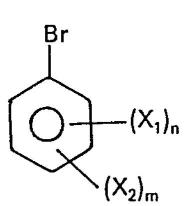

where $X_1$ and $X_2$, which are identical or different, stand for a chlorine atom or a fluorine atom, n and m are greater than or equal to 0 and smaller than or equal to 3, the sum n + m being equal to 2 or 3, an alkaline base and at least one compound forming a complex with the cation of the alkaline base.

2. A process according to Claim 1, characterised in that the compound forming a complex with the cation of the alkaline base is a macrocyclic polyether having from 15 to 30 atoms in the ring and consisting of 5 to 10 -O-X units in which X is either $-CHR_1-CHR_2-$ or $-CHR_1-CHR_4-CR_3R_2-$, $R_1$, $R_2$, $R_3$ and $R_4$, which are identical or different, being a hydrogen atom or an alkyl radical containing from 1 to 4 carbon atoms, one of the X being capable of being $-CHR_1-CHR_4-CR_3R_2-$ when the -O-X units incorporate the group $-O-CHR_1-CHR_2-$.

3. A process according to Claim 1, characterised in that the compound forming a complex with the cation of the alkaline base is a macrocyclic or bicyclic compound of the general formula Ia or Ib

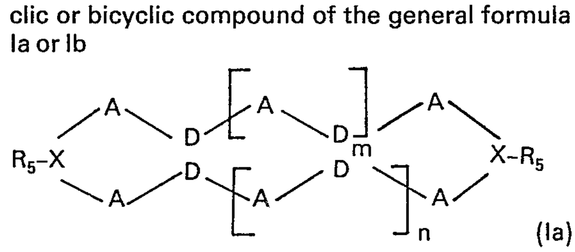
(Ia)

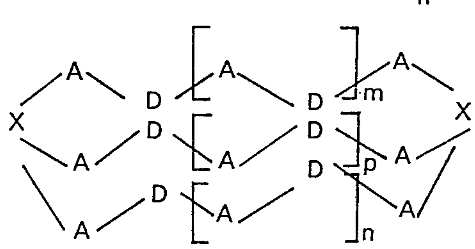
(Ib)

in which:

X stands for N or P,
A stands for an alkylene group containing from 1 to 3 carbon atoms,
D stands for O, S or N-$R_6$ where $R_6$ stands for an alkyl radical containing from 1 to 6 carbon atoms,
$R_5$ stands for an alkyl radical containing from 1 to 6 carbon atoms, and n, m and p, which are identical or different, are integers between 1 and 5.

4. A process according to Claim 1, characterised in that the compound forming a complex with the cation of the alkaline base is an amine of the general formula:

$$N-[CHR_7-CHR_8-O-(CHR_9-CHR_{10}-O)_s-R_{11}]_3 \quad (II)$$

in which s is an integer greater than or equal to 0 and smaller than or equal to 10 ($0 \leqq s \leqq 10$), $R_7$, $R_8$, $R_9$ and $R_{10}$, which are identical or different, stand for a hydrogen atom or an alkyl radical containing from 1 to 4 carbon atoms and $R_{11}$ stands for an alkyl or a cycloalkyl radical containing from 1 to 12 carbon atoms, a phenyl radical, or a radical $-C_qH_{2q}-\varnothing$ or $C_qH_{2q+1}-\varnothing-$, where q is between 1 and 12.

5. A process according to Claim 1, characterised in that compound forming a complex with the cation of the alkaline base is a linear polyether of the general formula (III)

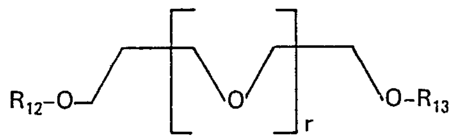
(III)

in which r is between approximately 1 and approximately 10 and where $R_{12}$ and $R_{13}$, which are identical or different, stand for an alkyl radical containing from 1 to 12 carbon atoms.

6. A process according to Claim 2, characterised in that the macrocyclic polyether is chosen from the group comprising:

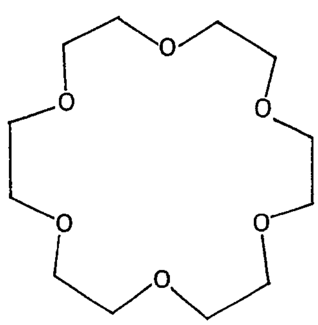

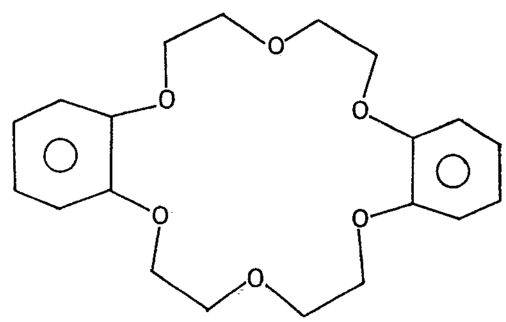

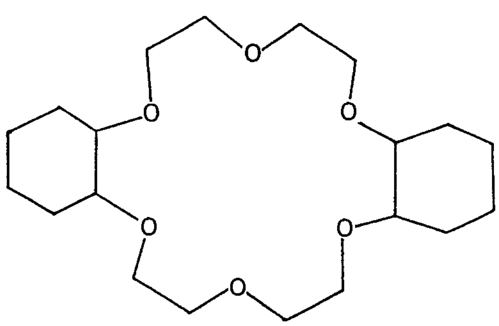

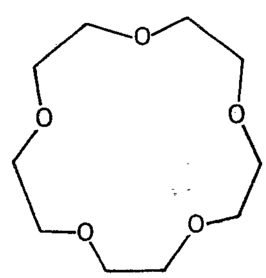

7. A process according to Claim 3, characterised in that the macrocyclic or bicyclic compound is chosen from the group comprising:

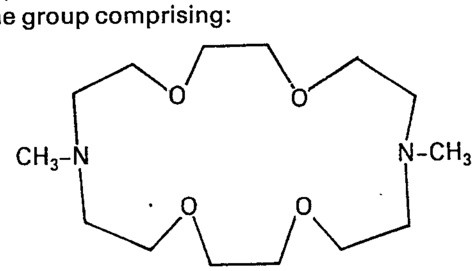

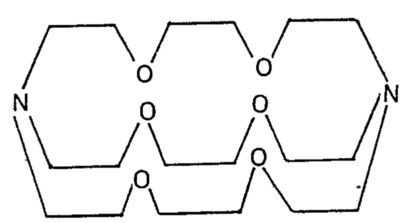

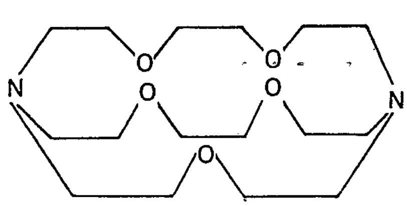

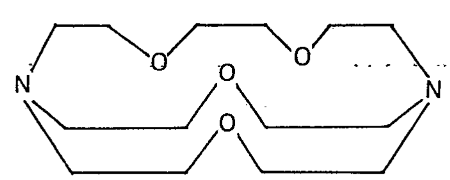

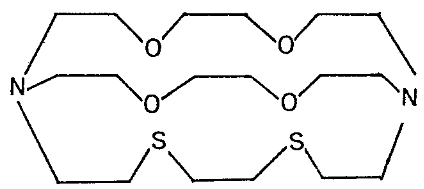

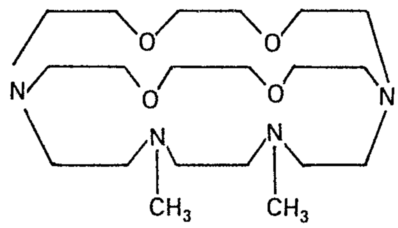

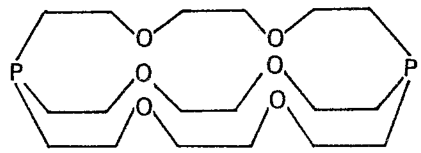

8. A process according to Claim 4, characterised in that in the formula II, $R_7$, $R_8$, $R_9$ and $R_{10}$ stand for a hydrogen atom or a methyl radical.

9. A process according to Claim 4, characterised in that in the formula II, s is greater than or equal to 0 and smaller than or equal to 6 and $R_{11}$ stands for an alkyl radical containing from 1 to 4 carbon atoms.

10. A process according to Claims 8 and 9, characterised in that the amine is chosen from the group comprising tris(3,6-dioxaheptyl)amine and tris(3,6-dioxahexyl)amine.

11. A process according to Claim 5, characterised in that the linear polyether is chosen from the group comprising

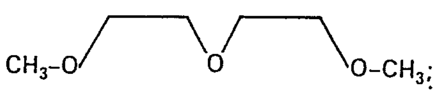;
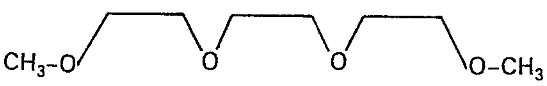

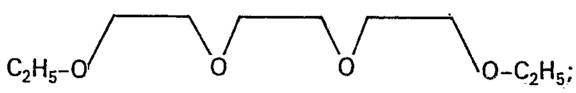;
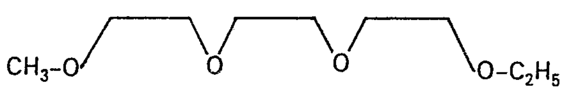

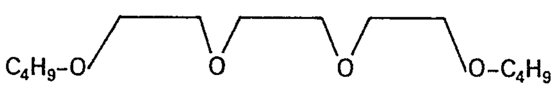

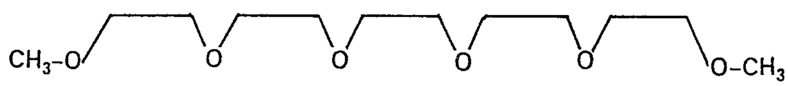

12. A process according to any one of the principal claims characterised in that the alkaline base is an alkali metal amide.

13. A process according to any one of the preceding claims, characterised in that the bromohalobenzene employed is 2,4-dichlorobromobenzene, 2,3-dichlorobromobenzene or 3,5-difluorobromobenzene.

14. A process according to any one of the preceding claims, characterised in that it is carried out in the presence of a non-polar aprotic solvent or a weakly polar aprotic solvent.

15. A process according to any one of the preceding claims, characterised in that the molar ratio of the alkaline base to the bromohalobenzene is between 0.05 and 1.

16. A process according to any one of the preceding claims, characterised in that the molar ratio of the compound forming a complex with the cation of the alkaline base is between 0.005 and 0.2.

17. A process according to any one of the preceding claims, characterised in that it is carried out at a temperature between 0 and 60°C.

**Patentansprüche**

1. Verfahren zur Isomerierung von Bromhalogenbenzolen, dadurch gekennzeichnet, dass man ein Bromhalogenbenzol der Formel

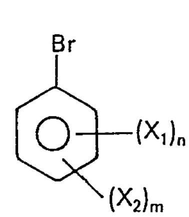

in der $X_1$ und $X_2$ gleich oder verschieden ein Chlor- oder Fluoratom darstellen, n und m grösser oder gleich 0 und kleiner oder gleich 3 sind, wobei die Summe n + m gleich 2 oder 3 ist, zusammen mit einer Alkali-Base und mindestens einer das Kation der Alkali-Base komplex bindenden Substanz einsetzt.

2. Verfahren nach Anspruch 1, dadurch gekennzeichnet, dass die Komplexverbindung für das Kation der Alkali-Base ein makrocyklischer

Polyäther mit 15 bis 30 Atomen im Ring ist und aus 5 bis 10 -O-X-Einheiten besteht, in denen X entweder $-CHR_1-CHR_2-$ oder $-CHR_1-CHR_4-CR_3R_2-$ ist, wobei $R_1$, $R_2$, $R_3$ und $R_4$ gleich oder verschieden ein Wasserstoffatom oder ein Alkylrest mit 1 bis 4 Kohlenstoffatomen sind und einer der Reste X $-CHR_1-CHR_4-CR_3R_2-$ sein kann, wenn die -O-X-Einheiten die Gruppierung $-O-CHR_1-CHR_2-$ enthalten.

3. Verfahren nach Anspruch 1, dadurch gekennzeichnet, dass die Komplexverbindung für das Kation der Alkali-Base eine makrocyklische oder bicyklische Verbindung der allgemeinen Formel Ia oder Ib ist

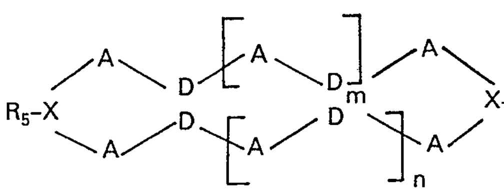

(Ia)

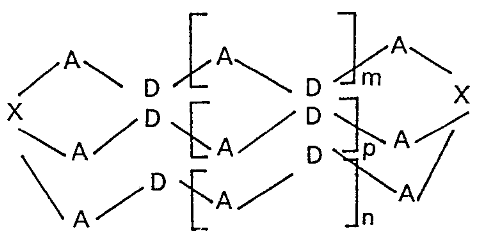

(Ib)

in denen:

- X N oder P darstellt,
- A eine Alkylengruppe mit 1 bis 3 Kohlenstoffatomen darstellt,
- D O, S oder $N-R_6$ darstellt, wobei $R_6$ einen Alkylrest mit 1 bis 6 Kohlenstoffatomen bedeutet,
- $R_5$ einen Alkylrest mit 1 bis 6 Kohlenstoffatomen darstellt, wobei n, m und p gleich oder verschieden ganze Zahlen zwischen 1 und 5 sind.

4. Verfahren nach Anspruch 1, dadurch gekennzeichnet, dass die Komplexverbindung für das Kation der Alkali-Base ein Amin der allgemeinen Formel

$$N-[CHR_7-CHR_8-O-(CHR_9-CHR_{10}-O)_s-R_{11}]_3 \quad (II)$$

ist, in der s eine ganze Zahl grösser oder gleich 0 und kleiner oder gleich 10 ($0 \leqq s \leqq 10$) ist, wobei $R_7$, $R_8$, $R_9$ und $R_{10}$ gleich oder verschieden ein Wasserstoffatom oder einen Alkylrest mit 1 bis 4 Kohlenstoffatomen bedeuten und $R_{11}$ einen Alkyl- oder Cykloalkylrest mit 1 bis 12 Kohlenstoffatomen, einen Phenyl- oder $-C_qH_{2q}-\varnothing-$ oder $C_qH_{2q+1}-\varnothing$-Rest darstellt, in dem q zwischen 1 und 12 liegt.

5. Verfahren nach Anspruch 1, dadurch gekennzeichnet, dass die Komplexverbindung für das Kation der Alkali-Base ein linearer Polyäther der allgemeinen Formel (III)

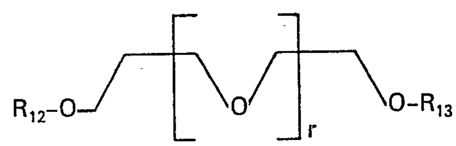

(III)

ist, in der r etwa 1 bis 10 beträgt und $R_{12}$ und $R_{13}$ identisch oder unterschiedlich, einen Alkylrest mit 1 bis 12 Kohlenstoffatomen bedeuten.

6. Verfahren nach Anspruch 2, dadurch gekennzeichnet, dass der makrocyklische Polyäther aus der Gruppe der folgenden Verbindungen gewählt ist:

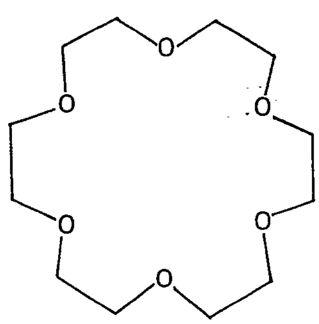

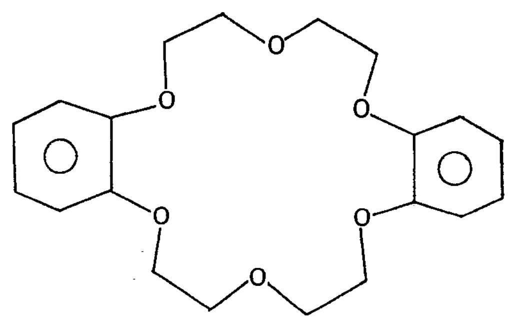

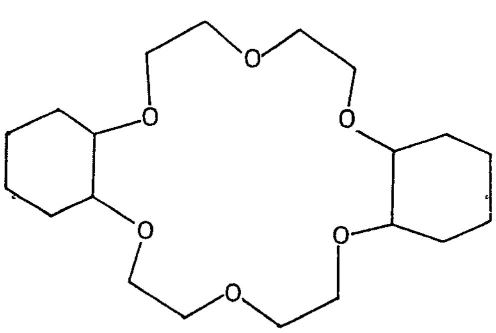

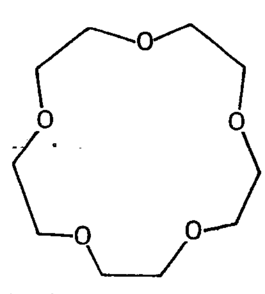

7. Verfahren nach Anspruch 3, dadurch gekennzeichnet, dass die makrocyklische oder bicyklische Verbindung aus der Gruppe der folgenden Verbindungen gewählt ist:

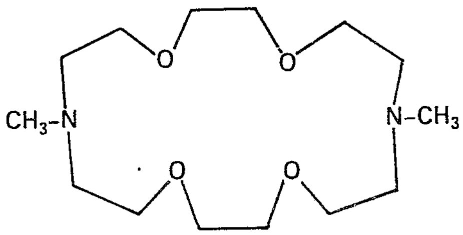

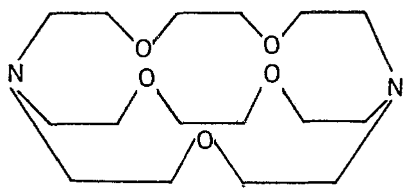

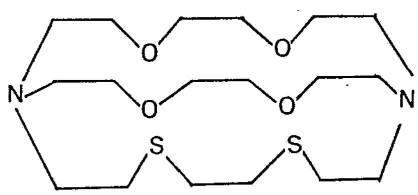

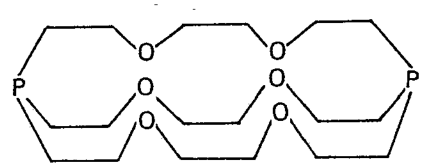

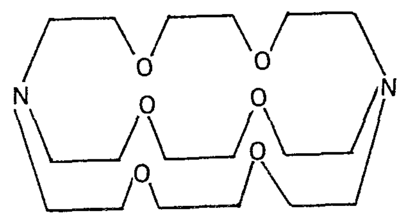

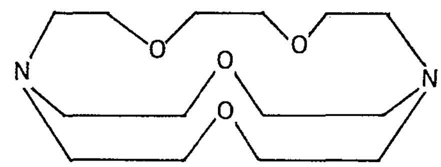

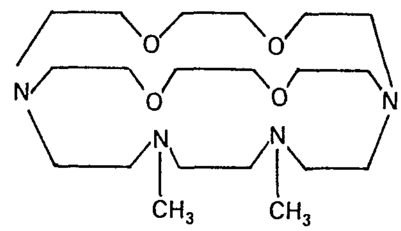

8. Verfahren nach Anspruch 4, dadurch gekennzeichnet, dass in der Formel II $R_7$, $R_8$ und $R_9$ ein Wasserstoffatom oder einen Methylrest bedeuten.

9. Verfahren nach Anspruch 4, dadurch gekennzeichnet, dass in der Formel II s grösser oder gleich 0 und kleiner oder gleich 6 ist und $R_{11}$ einen Alkylrest mit 1 bis 4 Kohlenstoffatomen bedeutet.

10. Verfahren nach den Ansprüchen 8 und 9, dadurch gekennzeichnet, dass das Amin aus der Gruppe von Tris(3,6-dioxaheptyl)amin und Tris(3,6-dioxahexyl)amin ausgewählt ist.

11. Verfahren nach Anspruch 5, dadurch gekennzeichnet, dass der lineare Polyäther unter einer der folgenden Verbindungen gewählt ist:

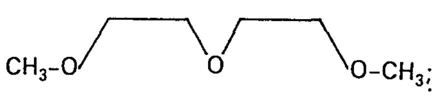;
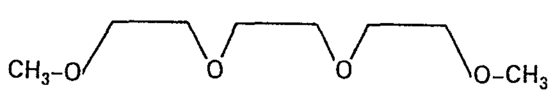

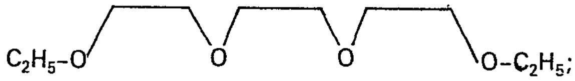;
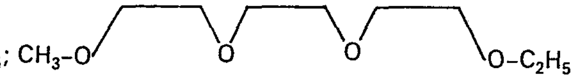

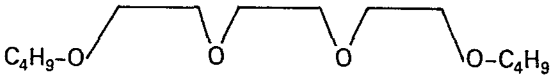

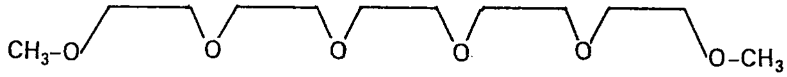

12. Verfahren nach einem der Hauptansprüche, dadurch gekennzeichnet, dass die Alkali-Base ein Alkaliamid ist.

13. Verfahren nach einem der vorangehenden Ansprüche, dadurch gekennzeichnet, dass das eingesetzte Bromhalogenbenzol 2,4-Dichlor-brombenzol, 2,3-Dichlor-brombenzol oder 3,5-Difluor-brombenzol ist.

14. Verfahren nach einem der vorangehenden Ansprüche, dadurch gekennzeichnet, dass man in Anwesenheit eines aprotischen apolaren oder wenig polaren Lösungsmittels arbeitet.

15. Verfahren nach einem der vorangehenden Ansprüche, dadurch gekennzeichnet, dass das Molverhältnis der Alkali-Base zum Bromhalogenbenzol zwischen 0,05 und 1 liegt.

16. Verfahren nach einem der vorangehenden Ansprüche, dadurch gekennzeichnet, dass das Molverhältnis der Komplexverbindung für das Kation der Alkali-Base zwischen 0,005 und 2 liegt.

17. Verfahren nach einem der vorangehenden Ansprüche, dadurch gekennzeichnet, dass man bei einer Temperatur zwischen 0 und 60°C arbeitet.